(19) Europäisches Patentamt

European Patent Office

Office européen des brevets

(11) Publication number: **0 270 976 B1**

# EUROPEAN PATENT SPECIFICATION

(45) Date of publication of patent specification: **25.08.93**  (51) Int. Cl.⁵: **A61F  13/15**

(21) Application number: **87117694.7**

(22) Date of filing: **30.11.87**

(54) **A process and apparatus for forming a product comprising, in part or as a whole, a wad of finely divided material.**

(30) Priority: **02.12.86 ZA 869098**
        **14.09.87 ZA 876875**

(43) Date of publication of application:
     **15.06.88 Bulletin  88/24**

(45) Publication of the grant of the patent:
     **25.08.93 Bulletin  93/34**

(84) Designated Contracting States:
     **AT BE CH DE ES FR GB GR IT LI LU NL**

(56) References cited:
     **WO-A-85/04366      DE-A- 2 918 595**
     **DE-A- 3 614 969     FR-A- 2 229 383**
     **US-A- 1 950 765     US-A- 2 949 646**
     **US-A- 3 994 047**

(73) Proprietor: **McNEIL-PPC, INC.**
     **Van Liew Avenue**
     **Milltown New Jersey 08850(US)**

(72) Inventor: **Levy, David George**
     **Summerhille Le Seuer Avenue**
     **Constantia 7800 Cape Province(ZA)**
     Inventor: **Oatlea, John Albert**
     **27 Franklin Crescent**
     **Eastward Ho 5256 Cape Province(ZA)**
     Inventor: **Sharp, William Bernard**
     **2 Van Der Poll Rd**
     **Takai 7899 Cape Province(ZA)**

(74) Representative: **Strehl Schübel-Hopf Groening**
     **& Partner**
     **Maximilianstrasse 54 Postfach 22 14 55**
     **D-80504 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

TECHNICAL FIELD

THIS INVENTION relates to a process and apparatus for forming a product comprising in part or as a whole, a wad of finely divided materials, eg. a pad for absorbing body fluids.

BACKGROUND ART

Absorbent pads for use in absorbing body fluids are well-known. Such pads include baby diapers, sanitary napkins, incontinence pads, wound dressings, breast pads for nursing mothers and the like. They are commonly made of absorbent cellulosic pulp fibres in the form of a batt or wad sandwiched between at least two layers of material. One layer is fluid-permeable and forms the layer adapted to face the wearer's body. The other layer conveniently is liquid impervious and forms the side which faces away from the person's body and protects the person's clothing from soiling.

Various processes are known in the art for forming absorbent pads of the abovementioned kind. Reference may be had to:

USA specifications 1,950,765 (Winter); 2,073,329 (Winter); 2,949,646 (Da Clark); 3,512,218 (Langden); 3,518,726 (Banks); 3,846,871 (Kolbach); 3,939,240 (Savich); 3,973,291 (Kolbach); 4,005,957 (Savich); 4,016,628 (Kolbach); 4,560,379 (Stemmler); 4,592,708 (Feist) and 4,598,441 (Stemmler); as well as European patent applications 0,151,033 (Petersen) and 0,175,774 (Johnson).

In summary, a first general type of process involves laying a continuous web of fibres onto a suitable transport mechanism such as a conveyor belt. The continuous web is then separated into discrete wads by cutting or the like before enclosing them in outer layers of material. USA specification No. 4,016,628 is an example of this type of process.

In a second general type of process, the wads are formed individually in pre-shaped pockets before transferring them onto one of the surrounding layers (the backing layer or the facing layer). Examples of machinery for enabling this second type of process to be carried out are illustrated in USA Patent Specifications Nos 4,005,957; 4,592,708; 3,973,291; 3,846,871; 3,939,240; 3,518,726; 4,560,379; and 4,598,441; and the abovementioned two European Patent Specifications. In these specifications, the absorbent pulp fibres are formed into a wad of a predetermined shape in the pocket. If desired, fibres of more than one absorbency can be utilized for forming the wad.

In the apparatus used for carrying out this second general type of process, various modifications are possible in the manner in which the material for forming the wads (finely divided fibres of wood or the like) is transported in a fluid (usually air) into the pre-shaped pockets. Also, the shapes of the pockets and/or their depth at different position may be varied.

For example, in one apparatus the fibres, suspended in air, enter the curved surface of a stationary drum at the top and leave it through an aperture at the bottom to rain down into the pockets on a conveyor belt. In another apparatus, the drum may rotate and the pockets can be apertures in the curved surface of the exterior of the drum, with the fibres raining down on that surface. In yet another apparatus, the rotating drum is effectively replaced by a continuous conveyor belt with the pockets formed in it. All of the apparatus have the stream of air and fibres entering the apparatus and move directly towards the pockets in substantially a straight line.

This second general type of process has many advantages over the first type in that hard edges, (caused by cutting in the first type) are avoided. Furthermore, shaped and tapered pads may be formed with layered constructions if desired. The different layers can have different absorbent properties.

The second general type of process also has disadvantages. Intricate wad designs, most suitable for comfortable application to the human body are limited by the need to transfer the friable fibre wad from the forming pockets onto a moving substrate. The position of the formed wads in relation to the other materials used in the manufacture of the product often varies when the transfer process takes place. The wad may lose it's shape and layers of different fibre types may become dislodged from each other during transfer.

In a third general type of process discrete pads are formed directly onto a fluid permeable layer without the necessity of having a transfer step from pre-shaped pockets. USA Specifications No 1,950,765 and No. 2,073,329 (both of C.P. Winter) illustrate this general type of process and USA specification No. 2,949,646 (D.A. Clark) discloses an apparatus which could operate in a similar manner. This general type of process has advantages but its scope is limited. We have developed a new and inventive process and apparatus of the third general type which has advantages over the prior art.

## DISCLOSURE OF THE INVENTION

It would be desirable to provide a process and apparatus in which the wads are formed at pre-determined positions on a substrate, and using an apparatus with an inlet system such that the amount and/or positioning of the fibres can be controlled and/or varied.

The present invention provides a process for the manufacture of a product comprising in part or as a whole a wad of finely divided materials, which process comprises the steps of:

(a) providing a former having a plurality of apertures therein;

(b) supplying a movable continuous band of fluid-permeable material to one side of the former to close off that side of the apertures therein in a fluid-permeable manner;

(c) applying a fluid pressure difference across the former to cause a lower pressure side to be formed on one side of the former and a higher pressure side on the other side of the former, with a fluid flow through the apertures and through the fluid permeable material which closes off the apertures;

(d) introducing at least two inlet streams of fluid into the higher pressure side from opposite sides transversely of the direction of movement of the continuous band, at least one of said streams containing finely divided materials suspended therein;

(e) permitting the finely divided materials to pass into the apertures and be deposited on the continuous band of fluid-permeable material;

(f) and causing the band of fluid-permeable material with the wad of finely divided materials thereon to be separated from the former.

The invention also provides an apparatus for the manufacture of a product comprising, in part or as a whole, a wad of finely divided materials, which apparatus comprises a wad-forming station having former with a plurality of apertures formed therein, a system adapted to supply a band of fluid-permeable material along one side of the former to close off that side of the apertures therein in a fluid-permeable manner, said former and band of fluid permeable material being positioned between a first side of the wad-forming station which is to be at a higher pressure than a second side which is to be at a lower pressure, a fluid inlet into the first side of the wad-forming station and positioned transversely of the direction of movement of the band of fluid-permeable material, at least one further fluid inlet into the said first side of the wad-forming station and facing the first inlet from the opposite transverse side, means for supplying finely divided materials to at least one of the fluid inlets, means for providing a pressure difference between the first and second sides of the wad-forming station, whereby the finely divided materials are caused to move into the apertures in the former and be deposited on the band of fluid permeable material.

With the present invention, the abovementioned prior art disadvantages of utilizing a pocket forming process are reduced or avoided. One is able to mix different material types of finely-divided material if desired and to lay them in predetermined and intricate shapes as desired directly onto the fluid-permeable band. The fluid-permeable band conveniently may be a vapour permeable but substantially liquid-impervious material. If desired, the side of the vapour-permeable substantially liquid-impervious material which faces the higher pressure side of the former may have water on it, eg. by spraying. In this embodiment, pressure may thereafter be applied to the wad to form a thin densified layer. Alternatively, the side of the fluid-permeable material facing the higher pressure side of the former may have adhesive on it. A suitable adhesive applicator may be provided in this embodiment.

The band may be the layer of material which ultimately is intended to be positioned against the wearer's body. This avoids the problem of having to transfer a pre-formed absorbent wad of fibres into a backing material. If desired such a band can be in contact with a fluid-permeable conveyor belt. Alternatively, the layer of fluid permeable material may be a fluid permeable conveyor belt itself.

With the present invention, the finely divided materials, suspended in a fluid (such as air) enter the wad-forming station from a direction which is transverse of the direction of movement of the fluid-permeable material. There is always at least one inlet through which the finely divided materials suspended in the fluid may pass. There is also a facing transverse fluid inlet which may be permanently open, or partially but not completely closed and which faces the first inlet from the opposite transverse side. If desired, further inlets may be provided as well. Appropriate means can be provided for independently adjusting the size of the inlets and/or the fluid flow rate through the inlets.

Conveniently, to manufacture a substantially uniform pad, both facing transverse inlets can be open to substantially the same amount and fluid containing finely divided materials may flow from a pair of opposite sides into a space in the higher pressure side of the wad forming station. Alternatively, only fluid may flow through one inlet, and both fluid and finely divided materials may flow through the other inlet. The rate of flow of fluid from the opposite transverse sides may be the same or different. Means may therefore be provided for adjusting the flow of fluid (eg. air). By adjusting the fluid stream flow rates, one or other side of the wads may be built up, or the wad can be of uniform thickness throughout.

By experimenting with the rate of flow of fluid from one or both sides, and/or by increasing the number of fluid inlets on one or both sides from a single inlet to a plurality of inlets, the effect of changing fluid flow patterns can be examined. It is then possible to select a desired inlet size and/or number of inlets on opposing transverse sides to give a desired depositing of the finely divided materials. Since the inlets face each other in a direction across the direction of movement of the fluid-permeable material, and need not directly face the fluid-permeable band, a fluid turbulence exists on the higher pressure side of the wad-forming station.

With the present invention, the side spacing of the inlet(s) causes the finely divided materials to be transported first in a direction in a plane which is not perpendicular to the base of the apertures, and secondly towards the base of the apertures. The fluid flow through an aperture of the former can vary from high fluid flow to low fluid flow depending on the position of the aperture in relation to the position of the inlets and outlet(s) for the fluid and/or the fluid flow through the inlets and outlet(s).

The surface of the wad facing the higher pressure side of the former may be subjected to different fluid flow in different areas to give a desired shape. Alternatively, the fluid flow on the surface facing the higher pressure side of the former may be substantially constant across the surface of the pad which is being formed.

Both flat and contoured wads may be manufactured with the apparatus provided by the invention. Wads of differing densities can also be manufactured. Wads can be manufactured which have different densities at different positions through the thickness. Adjustment of the rate of fluid flow, the period of flow, and/or the positions of the inlets and outlet can enable these wads to be manufactured.

The finely divided materials may be fibres of any suitable absorbent material. A single type of fibres may be used. Alternatively different fibres can be used as desired in the present process. Examples are various cellulosic fibres, eg wood, pulp, cotton, etc.

The interior of the former may be divided into at least two sections if it is desired to form a multilayered wad. A pair of streams of fluid, at least one of which contains finely divided materials suspended therein enters each section. The finely-divided material entering at least one section may differ from the material entering another section, so that layers of different composition may be built up. The passage of fluid from at least one of the sections through the former may, if desired, be restricted. A sandwich pad, which can have a central layer of smaller cross-sectional area, can be manufactured in this manner.

The former conveniently has apertures of predetermined shape corresponding to the shape of the absorbent wad to be formed. Thus, the former may have apertures of regular shape, eg. circular, oval, rounded rectangular, or trapezoid, on non-regular shape, eg. C-shape or dumbell shape, or other suitable shape formed therein. The former conveniently is a ring which may have inwardly-directed side flanges, as in a drum. Alternatively, the former can be a band of appropriate thickness having the apertures of desired shape formed therein.

A mill may be provided for milling raw wood pulp, super-absorbent fibres, staple fibres and/or powders to form the finely divided materials. These finely divided materials will form the wad of the final pad. Conveniently, the finely divided materials are supplied to the wad forming work station by means of pipes. Air or other suitable gas conveniently may be used as the fluid for supplying the finely divided materials to the wad forming station. The finely divided materials enter the wad-forming station through the inlets transversely of the direction of movement of the fluid-permeable material, ie from opposite sides of the apparatus. Conveniently, there are a plurality of inlets to an enclosed wad-forming station. We have found it convenient to have a generally box-like chamber as the wad forming station with the finely divided materials entering the box-like chamber transversely from a pair of opposite sides. It is also possible to have a former, eg. a ring-shaped former, moving between two box-like chambers.

The inlets, i.e. the pipes carrying the finely divided materials, enter an interior chamber transversely within the former. The interior chamber is then open at the surface abutting onto that part of the former towards which the finely divided materials move, and is substantially closed along the other sides. An exterior box-like chamber can be open at the side facing the interior chamber so that the former can rotate between the interior chamber and the exterior chamber. In this embodiment, the interior chamber is a high pressure chamber and the exterior box-like container is a lower pressure chamber.

In a second embodiment, a linear former can move between two chambers which are open along their facing sides and closed (apart from inlets and outlets) along their remaining sides. The two chambers may be of the same size, and may have no chamber which is an "inner" chamber with respect to the other.

As mentioned above, the former conveniently is a ring having the apertures of predetermined shape formed around its circumference. The inlets for the finely divided materials then may lead transversely to the inner chamber within the former. The fluid-permeable band passes around the exterior of the former and leads off to the next station. A rotary, or other movable brush can be provided, eg. inside the ring-like former, for enabling loose material, exceeding the desired amount in each aperture, to be brushed away. The loose material can then be removed from the interior of the former by means of vacuum applied through a vacuum outlet pipe.

Conveniently, the pressure difference across the apertures of the former is provided by applying a vacuum to the exterior of the outer box-like chamber, thereby causing the wad of finely divided material to move in the fluid stream into the aperture and be held against the fluid-permeable layer. Appropriate drives can be provided for driving the former and the movable brush.

The continuous band of fluid-permeable backing material (eg of cotton, paper, or other suitable fabric), with the predetermined shaped wads already formed thereon at spaced apart positions then passes to the next station. Vacuum may be continued to be applied beneath the fluid-permeable backing material to maintain the formed wads in position. The combination of the wad and backing layer can then be supplied to the next station where another layer forming the sandwich of the pads may be applied. Conveniently this comprises a fluid-impermeable layer to be applied over the pads. A polyethylene (e.g. "Surlyn") or other appropriate plastics material may be utilized as the fluid-impermeable layer. "Surlyn" is a Registered Trade Mark. If desired further layers may be applied over the fluid-impermeable layer. If desired the fluid permeable layer may be of sufficient width to be folded over at least the top of the wad. A liquid-permeable layer may be provided on the wad below the folded over portion.

Thereafter, the wad sandwiched or wrapped as described above can be supplied to a further station where sealing and/or embossing of the composite pads is carried out. The sealing conveniently is effected around the edge of the pads to seal the wad which will face the users body within the other external layers. Thereafter, a knife, which may be a rotary knife, is utilized to cut sealed composite pads from the external layers to form individual pads. The residue of the external layers can then be returned to a waste disposal unit for supply to waste-retrieval systems. The pads may then pass along a conveyor belt to a packaging station.

## BRIEF DESCRIPTION OF THE DRAWINGS

The invention is illustrated in non-limiting manner by reference to the accompanying drawings in which :
FIGURE 1 is a vertical view of one embodiment of an apparatus according to the invention; FIGURE 2 is a plan view of the apparatus of figure 1.
FIGURE 3 is a view of a former in the shape of a ring for use in the apparatus;
FIGURE 4 is a view of a second embodiment of the invention taken from a position which would be along line IV of Figure 1;
FIGURE 5 is a view of one embodiment of a wad forming station having a ring former; and
FIGURE 6 is a view of a third embodiment of an apparatus according to the invention, looking from the end of the apparatus to the ring; and
FIGURE 7 is a side view of the apparatus of Figure 1.
FIGURES 8a, 9a, 10a and 11a schematically show different arrangements for the entry of the supply fluid with fibres suspended therein;
FIGURES 8b, 9b, 10b and 11b illustrate the shape of deposits of fibres obtained in the apertures, following the arrangements of figures 8a through 11a respectively;
FIGURE 12 shows one preferred form of contoured wad that is obtained using the arrangement of figure 11a;
FIGURE 13 illustrates a second form of contoured wad that can be produced with the invention.
FIGURE 14 is a cross-section of a multilayered wad formed according to the invention; and
FIGURE 15 is a vertical section through an apparatus accordingly to the invention for making the wad of figure 14.

## DETAILED DESCRIPITON OF THE DRAWINGS

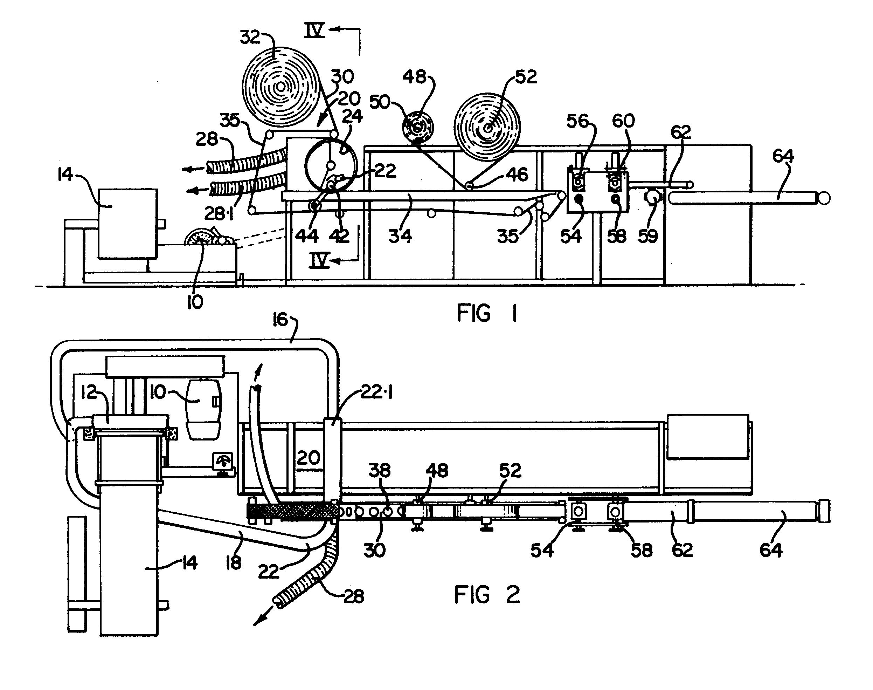

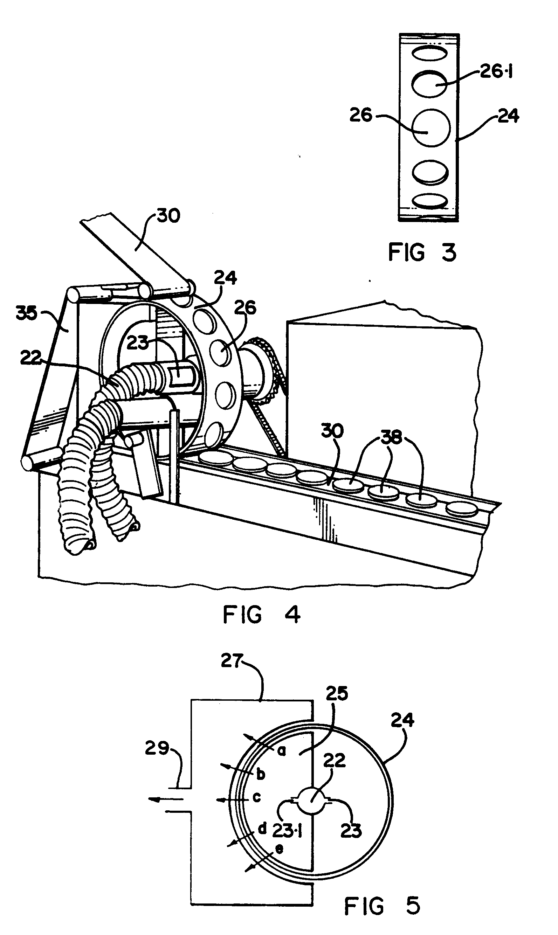

In Figures 1 to 5, a motor shown generally at 10 drives a mill 12. Raw pulp is supplied to the mill from a feed 14. The pulp is any suitable pulp, eg. wood or other fibres. The finely-divided pulp so formed passes along supply pipes 16, 18 together with air as the conveying fluid to enter a wad-forming station shown generally at 20 in the shape of a forming box. The pipes enter the station transversely and from opposing sides at 22, 22.1 into the interior of a ring 24 as shown in more detail in Figure 5. In Figure 1, both pipes 16, 18 carry fibres suspended in air. In Figure 4 only one large transverse pipe 22 is provided to carry air with fibres suspended in it, while a smaller transverse pipe (not visible) permits air to enter from the opposing side.

A plan view of the ring 24 is illustrated in Figure 3 and, as can be seen, it contains a plurality of apertures 26, 26.1 etc. passing through the circumference of the ring. The apertures are circular for forming breast pads but rings with other shaped apertures can be provided. The station 20, has the fibres entering from both the left and right sides at 22 and 22.1. An air inlet port shown at 23 (see Figure 4) is provided immediately in front of the pipes 16, 18. Air outlet pipes 28, 28.1 are provided at the back of the station 20. A vacuum is applied through the pipes 28, 28.1 to enable a pressure difference to arise between the inside of the ring where the finely divided pulp is supplied through the pipes 16, 18, and the outside of the ring which is covered by a continuous air-permeable liquid-pervious band 30 of fabric from supply reel 32. This continuous band, which will form the backing layer, passes around the outside of the ring 24 on a continuous fluid permeable supporting belt 35. The band 30 is then taken away from the ring 24 on the supporting belt 35. Vacuum may be applied below the supporting belt 35 at vacuum chamber 34 to maintain the formed wads in position on the air permeable band 30.

In the station 20, the band 30 of air-permeable material closes off one side of the apertures 26, 26.1 etc. whereby the fibres of finely divided material move into the apertures and form a predetermined shape of wad on the band of what is now backing layer 30. By providing circular apertures in the ring, a circular wad 38 will be formed on the backing layer 30 as it passes through the station 20.

Provided in the interior of the ring 24 is a brush 42 driven by a pulley drive 44. Alternatively the brush can be positioned outside the ring. The brush 42 brushes off loose fibres which project above the required size of the wad to be formed. The thickness and shape of the wad may be adjusted by adjusting the flow of the pulp from one side. The effect on the thickness of the wad by reducing flow from one side causes a reduction on the other side. If the two fibre streams having different compositions are employed blending of the fibres is achieved. Thus, super-absorbent fibres may be mixed in the wad with normal long fibre wood pulp or short fibre wood pulps may be mixed with staple length thermoplastic fibres. Separate mills and inlet pipes may lead to the wad-forming station to provide such wads.

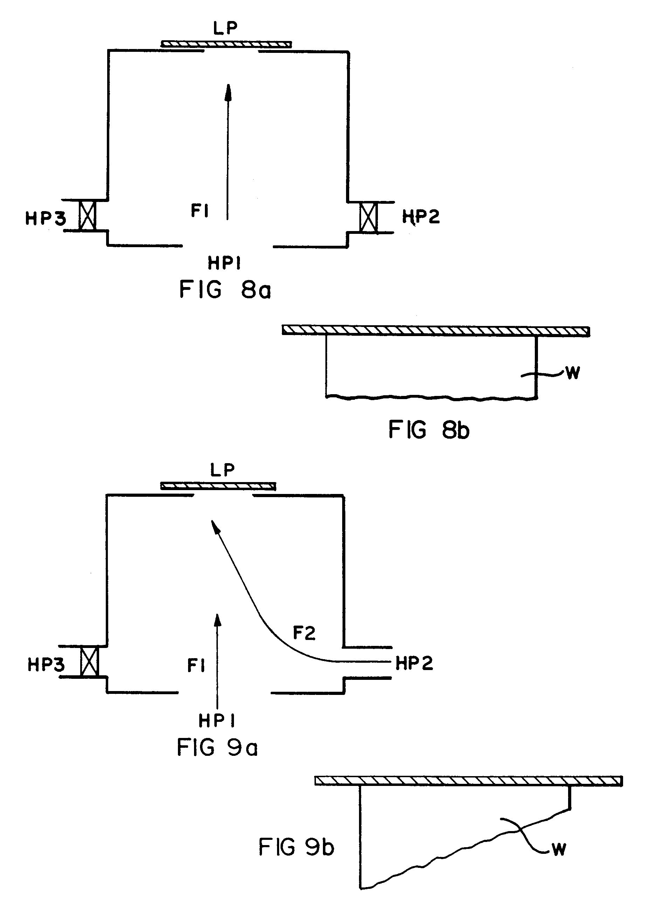

By adjusting the supply so that the amount, or size, or nature of the pulp is not the same from both fluid inlets, contoured wads can be obtained. This is explained more fully below by reference to figures 8 through 11. If wads of contoured thickness are to be formed, the height of a brush 42 can also have a contour to act at more than one level. Such wads are more fitting to the contours of the part of the human anatomy to receive such wads. This can make the absorbent pads formed from the wads more comfortable and effective for absorbing body fluids without leakage around the sides of pads.

The wads 38 on the backing layer 30, pass above the vacuum chamber 34 to a position 46 where a liquid-permeable layer 48 of Surlyn or other material is supplied from a reel 50. In addition, an additional backing layer is supplied from reel 52. The backing layer may be slip-resistant and/or have adhesive applied to it.

The sandwich comprising the fluid-pervious backing layer 30 with the shaped wads 38 on it and covered successively by a Surlyn layer 48 and backing layer are supplied to a station 54 where a sealer and embosser 56 seals the layers together. Thereafter, they pass to a station 58 where a rotary knife 60 cuts the sealed and embossed final absorbent pads from the continuous layers of material. The continuous layers, now with holes therein, pass around roller 59 and are sent to waste while the absorbent pads pass along vacuum conveyor belt 62 onto a further conveyor belt 64 from whence they are sent for packaging.

In Figure 5, the ring is shown at 24 the pulp inlet at 22 and air inlet port at 23. Inside the ring 24 is an inner chamber 25 which is open at its circumferential side. An outer chamber 27 is of lower pressure and air, together with fibres pass from 22 through opening 23.1 into inner chamber 25 and then through the apertures in the ring. The air then enters the outer chamber 27, and escapes through 29.

In experiments carried out with the apparatus, and illustrated below with reference to Figure 5, different results were obtained from wads formed at different points. For illustration, the wad-forming apertures 26 have been marked a, b, c, d and e to identify different positions. The machine was run at a speed of 110 wads per minute.

Ring dimensions:

The ring has 12 apertures at 113,09 mm centres,
    thus circumference is 1357,98 mm      = 1,36 m
    then diameter is 431,97 mm        = 0,43 m
    then radius is 215,99 mm         = 0,22 m
    aperture radius is 43 mm.
The chambers 25 and 27 compasses 5 apertures (a to e) Residence time within the chambers is therefore 5/110 min = 2,727 secs

$$\text{Linear machine speed} = 110 \times 113,09$$
$$= 12439,9 \text{ mm/min}$$
$$= 12,44 \text{ m/min.}$$

An experiment was carried out to measure pulp added on at various stages within the chambers using a rapid pulp cut-off. The pulp supply pipe was removed and the conveyor stopped after 5 additonal wads had exited the wad-forming station.

Results:

| Pocket No. | Pulp Mass (mean of 4) | Time within pulpstream | Pulp Add on/sec. | Pulp Add on (%) |
|---|---|---|---|---|
| a | 0,175 g | 0,55 sec | 0,318 g | 11 % |
| b | 0,625 g | 1,09 sec | 0,818 g | 29 % |
| c | 1,250 g | 1,64 sec | 1,136 g | 39 % |
| d | 1,450 g | 2,18 sec | 0,364 g | 13 % |
| e | 1,575 g | 2,73 sec | 0,227 g | 8 % |

The results above indicate that the pulp add on is greater at position (c). If pulp add on its set at 100 for position (c) then the other positions have pulp add on of 28(a) 73(b) 33(d) and 21(e). The pulp add on at (e) although less than (a) is surprisingly high indicating that any reduction in air flow by the partially formed wad is small compared to the effect of position of the aperture within the forming station. Positions generally adjacent the vacuum outlets were found to have the greatest pulp add on.

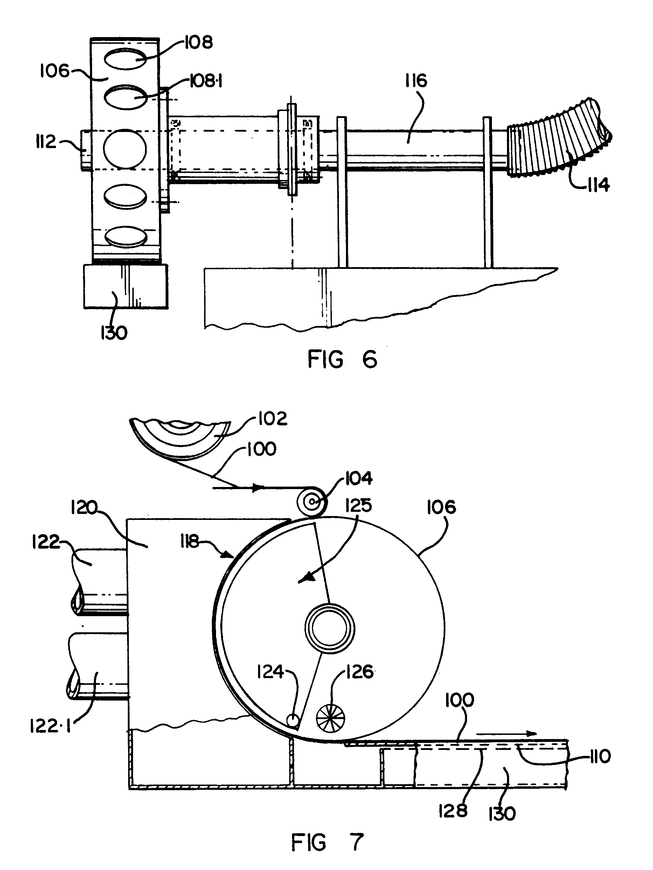

Referring now to Figures 6 and 7, a long band 100 of air permeable material passes from a stock 102 over roller 104 and around a ring 106 having twelve wad-forming apertures 108, 108.1 etc in its circumference. The band 100 passes along a continuous air pervious conveyor belt 110.

Air enters the interior of the ring 106 transversely of the movement of the band 100 from one side through axial inlet 112. Air with finely divided wood pulp suspended therein passes along flexible hose 114 into inlet pipe 116 and enters the ring axially from the other side. Thus the flow of air and pulp, and the flow of air into the ring is transversely of the direction of movement of the band 100.

A seal 118 is provided around the ring edge and a vacuum applied to vacuum box 120 through vacuum pipes 122 and 122.1. A free rotating roller 124 within inner chamber 125 reduces the leakage of air into the forming station at the most likely point of maximum leakage.

A brush 126 is provided for removing excess finely divided material. The continuous conveyor belt 110 runs on a perforated upper plate 128 of vacuum box 130 to assist in removing pulp from the aperture pockets of the ring by applying a vacuum to box 130.

An appropriate electrical panel, drive means and other suitable accessories can be provided.

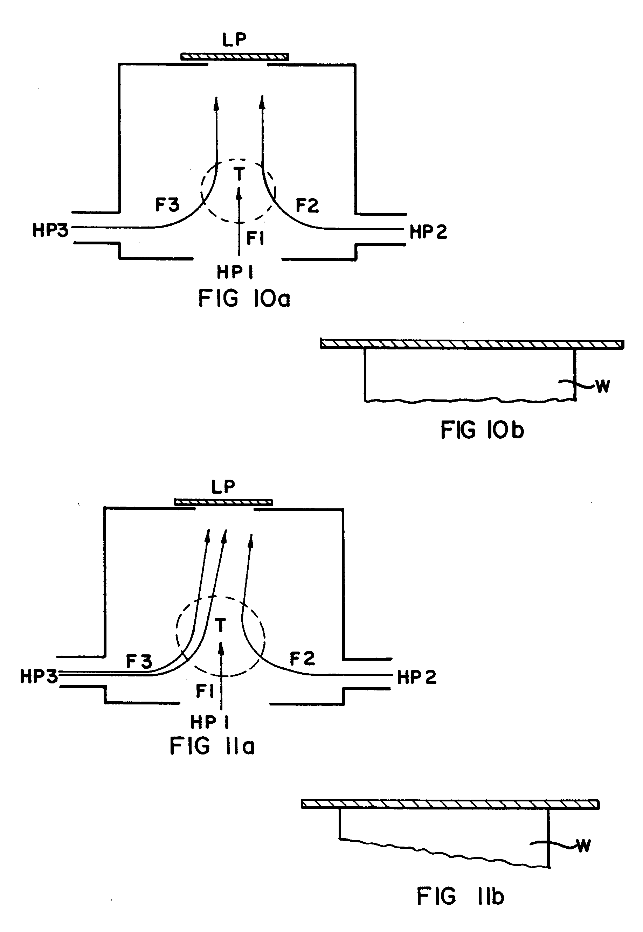

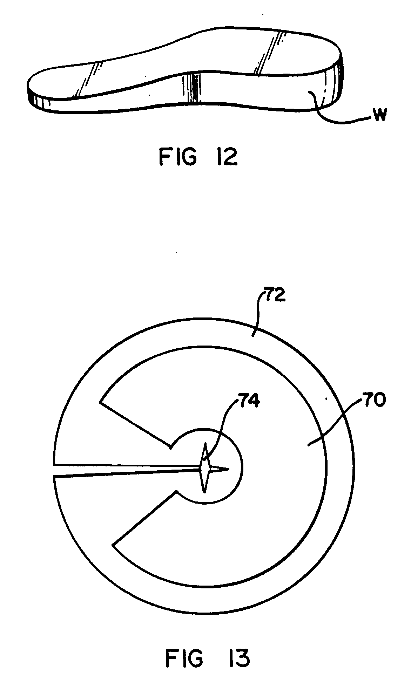

Figures 8 through 11 will now be described as they illustrate the effect of varying the fluid flow. Figures 8a and 9a are not in accordance with the invention but figures 10a and 11a are.

In figure 8a, there exists a fluid flow (F1) from a high pressure inlet (HP1) to low pressure outlet aperture (LP) i.e. aperture 26 of the ring 24 of figure 3. HP2 and HP3 are closed and are the inlets 22 and 22.1 of figure 2. A wad (W) of the shape shown in figure 8b is obtained if finely divided materials are introduced into fluid flow F1.

In figure 9a with high pressure inlet (HP2) now open fluid flow F2 is caused to change direction by fluid flow F1 towards aperture LP. In this situation it is found that finely divided materials entering at HP2 are deposited in the aperture LP as shown in cross section in figure 9b.

In figure 10a with high pressure inlet (HP3) also open, fluid flow F3 is also caused to change direction by F1 towards LP. If F2 and F3 are open by approximately equal amounts the resultant fluid flow is almost normal to the plane of LP and even thicknesses of finely divided materials are deposited as shown in cross section in figure 10b. Because of the turbulence at T it is not important (for single component wads)

7

whether the finely divided materials are supplied through HP2 or through both HP2 and HP3. If the wad comprises more than one component then separate finely divided materials can be supplied through separate high pressure inlets. Mixing of the two components can occur in the space where turbulence occurs.

In Fig 11a, F3 is open by a greater amount than F2 and the resultant fluid flow to LP is displaced from normal by the unequal flows. A wad of cross section 11b illustrates the deposition of the finely divided materials. This contoured deposition is particularly useful for anatomically conforming sanitary pads.

Figure 12 shows a typical shape for wad W obtained according to figure 11a.

The invention enables one to manufacture very stable absorbent pads because the wad defining the central absorbent layer is formed directly on one of the outer layers. If desired, an adhesive may be applied to the layer on the side which will face the pulp-airstream in the wad forming step. The pulp fibres, on being deposited on the fluid permeable layer become adhered to it, thus promoting the formation of an even more stable product. Instead of adhesive, water may be utilized, in which case the absorbent pulp contacting the water forms a surface of higher absorbency due to hydrogen bonding. This can be enhanced by later application of pressure.

The use of water to form a densified layer, is applicable to finely divided materials such as wood pulp fibres or cotton linters. In those embodiments fine droplets of water are applied to a batt of fibres and then the batt is compressed. A thin densified layer is thereby formed which has a greater absorbency rate than the batt immediately adjacent to it.

Water can be applied to the fluid permeable band or layer before the wad forming step. As the finely divided materials are deposited onto the fluid permeable band or layer they become wetted by the water. Subsequent application of pressure causes a thin densified layer to be formed.

Application of water in this manner has particular relevance when liquid impervious vapour permeable materials are used. The thin densified layer in this case would be positioned next to the liquid impervious layer in the finished product and provides greater absorbency by wicking body fluid away from the body facing layer and spreading it on the back of the wad. Thus the water does not bond the wad to the fluid permeable layer but provides wicking paths.

With the invention, one is able to form more intricate shapes. For example, one can make "doughnut shaped" surgical dressings for application to tracheostomy drains and the like by cutting a circular aperture with a segment retained. The incomplete disc so formed can be sealed between two layers. By subsequent cutting it can form an effective shaped dressing pad. Such intricate designs were not able to be achieved with the prior art processes known to us because the friable wad become broken or disturbed during transfer from the forming means onto the carrying belt. With the present invention, intricate pads can be formed in their final position on one of the outer layers of the product.

A surgical dressing of "doughnut shape" is illustrated in figure 13. This dressing comprises an absorbent wad 70 of the segmented doughnut shape illustrated. The wad 70 is formed using an aperture of generally C-shape. The wad 70 is applied to an outer layer 72 and a center cut 74 made for fitting around a wound drain. The wad 70 is formed directly on the outer layer 72, so no transfer step takes place. Although fig.13 illustrates a substantially round product it is understood that similar products with wads having substantially straight sides are also possible with the invention.

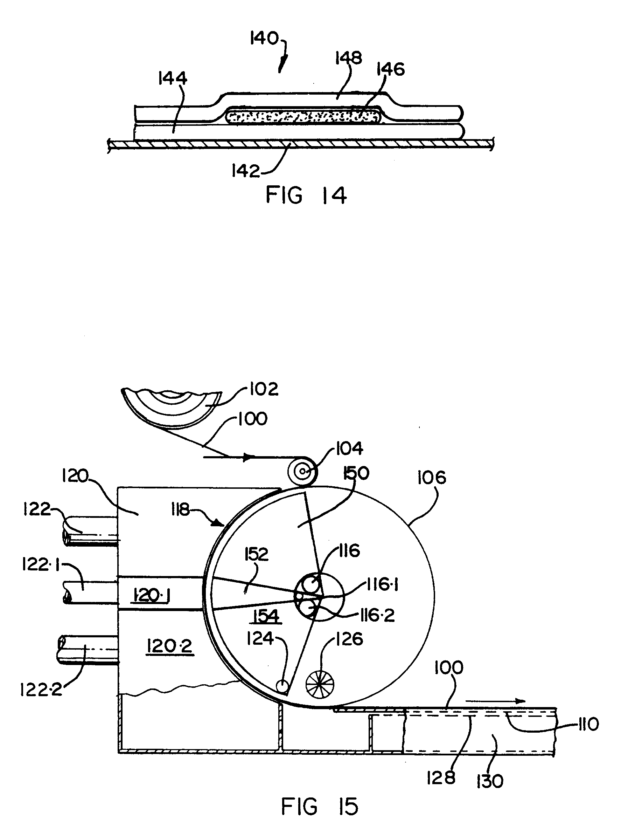

Referring now to figure 14, pad 140 comprises a fluid-permeable layer 142 on which has been deposited a first layer 144 of one type of finely divided material (f.d.m.1). A second layer 146 of a different finely divided material (f.d.m.2) is deposited on the first layer 144, and a third layer 148 of a still different finely divided material (f.d.m.3) is deposited on the other layers.

In figure 15, the apparatus is similar to that of figure 7 and similar numbering is used. A long band 100 of air permeable material passes from a stock 102 over rolled 104 and around a ring 106 containing twelve wad-forming apertures. The band 100 passes along a continuous air-pervious conveyor belt 110.

The interior of the ring 106 is divided into three forming stations 150, 152 and 154 which are separate chambers each with a separate pair of facing fluid inlets of which one inlet 116, 116.1 and 116.2 per chamber is visible. The stations 150, 152 and 154 are at a higher pressure than downstream vacuum boxes 120, 120.1 and 120.2. Vacuum pipes 122, 122.1 and 122.2 lead from each of the vacuum boxes 120, 120.1 and 120.2.

A free rotating roller is shown at 124 and a brush at 126. The band 100 runs on continuous conveyor belt 110, which passes over perforated upper plate 128 of vacuum box 130 in a similar manner to that illustrated in Figure 7.

The separate chambers forming the stations 150, 152 and 154 are open at their circumferential sides to permit the finely divided material passing through them to be deposited in the apertures. The central station 152 is only partially open at its circumferential side, thereby restricting deposition of the finely divided

EP 0 270 976 B1

material in the central portion of the apertures.

The invention has been described with reference to the fluid-permeable backing layer passing around the ring being a final layer for the product. If this backing layer is replaced by an air-permeable conveyor belt (eg. belt 35) one can supply a mixture of wood pulp fibres or other suitable finely divided material through one inlet and thermoplastic fibres or powder through another inlet to form thermoformable wads directly on the air-pervious conveyor belt. Subsequent thermal ultrasonic or other radiation treatment can bond the fibres together into a integral pad of the desired shape before removal from the conveyor belt. Thus, small wiping cloths suitable for cosmetic use, or filter packs for fluid filtration, may be made in this manner with the same process and apparatus.

Instead of having a single mill, a plurality of mills, eg. two or more can be utilized. Similarly a plurality of forming stations can be used eg. to provide multi-layered wads.

The apertures in the former may have vertical or contoured sides as desired.

In an embossing station, appropriate embossing to provide wicking can be incorporated. Any suitable wicking, eg. wicking to move fluid rapidly from the centre of the pad towards the outside can be embossed on the pads.

It is to be understood that the term "backing" has been used for convenience, and generally forms the body-facing layer in use. Other "backings" suitable for use in the process include liquid-impervious vapour-permeable materials such as spunbonded non-woven fabrics. When used in the process such materials serve as the "backing" in the process and as the backing of the finished pad.

**Claims**

1. A process for the manufacture of a product comprising, in part or as a whole, a wad of finely divided materials, which process comprises the steps of:

   (a) providing a former having a plurality of apertures therein;

   (b) supplying a movable continuous band of fluid-permeable material to one side of the former to close off that side of the apertures therein in a fluid-permeable manner;

   (c) applying a fluid pressure difference across the former to cause a lower pressure side to be formed on one side of the former and a higher pressure side on the other side of the former, with a fluid flow through the apertures and through the fluid-permeable material which closes off the apertures;

   (d) introducing a least two inlet streams of fluid into the higher pressure side from opposite sides transversely of the direction of movement of the continuous band, at least one of said streams containing finely divided materials suspended therein;

   (e) permitting the finely divided materials to pass into the apertures and be deposited on the continuous band of fluid-permeable material;

   (f) and causing the band of fluid-permeable material with the wad of finely divided materials thereon to be separated from the former.

2. A process as claimed in claim 1 wherein the former is positioned in a box-like chamber and wherein two streams of fluid enter the box-like chamber from opposite sides.

3. A process as claimed in claim 2, wherein both streams of fluid are continuous.

4. A process as claimed in claim 1, wherein the former is positioned in a box-like chamber, the interior of the box-like chamber is divided into at least two sections and a pair of streams of fluid enter each section.

5. A process as claimed in claim 4, wherein the passage of fluid from at least one of the sections is restricted.

6. A process as claimed in any of claims 2 to 4, wherein both streams of fluid contain finely divided materials suspended therein.

7. A process as claimed in any of the preceding claims, wherein the fluid is air.

8. A process as claimed in any of claims 2 to 7, wherein the two streams of fluid have different flow rates.

9

**9.** A process as claimed in any of the preceding claims, wherein the band of fluid-permeable material is vapour permeable but substantially liquid-impervious.

**10.** A process as claimed in claim 9 wherein the side of the vapour-permeable liquid-impervious material which faces the higher pressure side of the former has water on it.

**11.** A process as claimed in claim 10 wherein pressure is thereafter applied to the wad to form a thin densified layer.

**12.** A process as claimed in any of claims 2 to 9 wherein the side of the fluid-permeable material facing the higher pressure side of the former has adhesive on it.

**13.** A process as claimed in any of the preceding claims, wherein a liquid impervious layer is thereafter applied over the wads.

**14.** An apparatus for the manufacture of a product comprising, in part or as a whole a wad of finely divided materials, said apparatus comprising a wad-forming station (20) having a former (24') with a plurality of apertures (26; 108) formed therein, a system adapted to supply a band (30; 100) of fluid-permeable material along one side of the former (24) to close off that side of the apertures (26; 108) therein in a fluid permeable manner, said former (24) and band (30; 100) of fluid-permeable material being positioned between a first side of the wad-forming station (20) which is to be at a higher pressure than a second side which is to be at a lower pressure, a fluid inlet (22; 116) into the first side of the wad-forming station (20) and positioned transversely of the direction of movement of the band (30; 100) of fluid-permeable material, at least one further fluid inlet into the said first side of the wad-forming station (20) and facing the first inlet (22, 116) from the opposite transverse side, means (10, 12, 14, 16, 18) for supplying finely divided materials to at least one of the fluid inlets, means (28; 120, 122) for providing a pressure difference between the first and second sides of the wad-forming station (20), whereby the finely divided materials are caused to move into the apertures (26; 108) in the former (24) and be deposited on the band (30; 100) of fluid-permeable material.

**15.** An apparatus as claimed in claim 14, wherein the further fluid inlets are permanently open.

**16.** An apparatus as claimed in claim 14, wherein the further fluid inlets have means for adjusting the sizes of the further inlets and/or the rates of flow of fluid through them.

**17.** An apparatus as claimed in any of claims 14 to 16, wherein the former (24) is a ring.

**18.** An apparatus as claimed in any of claims 14 to 17 wherein the former (24) is positioned between an interior higher pressure chamber and an exterior lower pressure chamber.

**19.** An apparatus as claimed in any of claims 14 to 18, wherein the former (24) is a linear former.

**20.** An apparatus as claimed in any of claims 14 to 19, wherein the apertures (26; 108) are C-shaped.

**Patentansprüche**

**1.** Verfahren zur Herstellung eines Erzeugnisses, bestehend teilweise oder vollständig aus einem Kissen feinverteilten Materials, wobei das Verfahren die Schritte umfaßt:
(a) Bereitstellen eines Formers mit mehreren Öffnungen darin;
(b) Zuführen eines beweglichen, fortlaufenden Bandes aus flüssigkeitsdurchlässigem Material zu einer Seite des Formers, um die Seite der darin vorhandenen Öffnungen in einer flüssigkeitsdurchlässigen Weise zu verschließen;
(c) Anlegen einer Flüssigkeitsdruckdifferenz an den Former, so daß eine Seite niedrigeren Druckes an einer Seite des Formers und eine Seite höheren Druckes an der anderen Seite des Formers gebildet wird, wobei ein Flüssigkeitsstrom durch die Öffnungen und durch das flüssigkeitsdurchlässige Material geleitet wird, das die Öffnungen verschließt;
(d) Einleiten von mindestens zwei Einlaß-Flüssigkeitsströmen in die Seite höheren Druckes von gegenüberliegenden Seiten quer zur Bewegungsrichtung des fortlaufenden Bandes, wobei mindestens einer der genannten Ströme feinverteiltes, darin suspendiertes Material enthält;

(e) Hineinleiten des feinverteilten Materials in die Öffnungen und Ablegen auf dem fortlaufenden Band des flüssigkeitsdurchlässigen Materials;

(f) und Trennen des Bandes aus flüssigkeitsdurchlässigem Material mit dem darauf angeordneten Kissen aus feinverteiltem Material von dem Former.

2. Verfahren nach Anspruch 1, bei dem der Former in einer behälterförmigen Kammer positioniert wird und wobei zwei Flüssigkeitsströme in die behälterförmige Kammer von gegenüberliegenden Seiten eingeleitet werden.

3. Verfahren nach Anspruch 2, wobei beide Flüssigkeitsströme kontinuierlich sind.

4. Verfahren nach Anspruch 1, bei dem der Former in einer behälterförmigen Kammer positioniert wird, wobei das Innere der behälterförmigen Kammer in mindestens zwei Abschnitte unterteilt ist und ein Paar Flüssigkeitsströme in jeden Abschnitt eingeleitet wird.

5. Verfahren nach Anspruch 4, bei dem der Flüssigkeitsdurchgang aus mindestens einem der Abschnitte gedrosselt wird.

6. Verfahren nach einem der Ansprüche 2 bis 4, bei dem beide Flüssigkeitsströme feinverteiltes, darin suspendiertes Material enthalten.

7. Verfahren nach einem der vorhergehenden Ansprüche, bei dem die Flüssigkeit Luft ist.

8. Verfahren nach einem der Ansprüche 2 bis 7, bei dem die beiden Flüssigkeitsströme unterschiedliche Strömungsgeschwindigkeiten aufweisen.

9. Verfahren nach einem der vorhergehenden Ansprüche, wobei das Band aus flüssigkeitsdurchlässigem Material dampfdurchlässig, aber im wesentlichen flüssigkeitsundurchlässig ist.

10. Verfahren nach Anspruch 9, bei dem Wasser auf der Seite des dampfdurchlässigen, flüssigkeitsundurchlässigen Materials vorhanden ist, die der Seite höheren Drucks des Formers zugekehrt ist.

11. Verfahren nach Anspruch 10, wobei danach Druck auf das Kissen ausgeübt wird, um eine dünne, verdichtete Schicht zu bilden.

12. Verfahren nach einem der Ansprüche 2 bis 9, bei dem die Seite des flüssigkeitsdurchlässigen Materials, die der Seite höheren Drucks des Formers zugekehrt ist, Klebstoff aufweist.

13. Verfahren nach einem der vorhergehenden Ansprüche, wobei die flüssigkeitsundurchlässige Schicht anschließend auf die Kissen aufgebracht wird.

14. Vorrichtung zur Herstellung eines Erzeugnisses, bestehend teilweise oder ganz aus einem Kissen feinverteilten Materials, wobei die Vorrichtung umfaßt eine Kissenformstation (20) mit einem Former (24'), wobei darin mehrere Öffnungen (26; 108) vorhanden sind, einem System, das zur Zufuhr eines Bandes (30; 100) aus flüssigkeitsdurchlässigem Material entlang einer Seite des Formers (24) geeignet ist, um diejenige Seite der darin vorhandenen Öffnungen (26; 108) in einer flüssigkeitsdurchlässigen Weise abzuschließen, wobei der genannte Former (24) und das Band (30; 100) aus flüssigkeitsdurchlässigem Material zwischen einer ersten Seite der Kissenformstation (20) positioniert sind, die unter höherem Druck steht als eine zweite Seite, die unter niedrigerem Druck steht, einem Flüssigkeitseinlaß (22; 116) in die erste Seite der Kissenformstation (20), der quer zur Bewegungsrichtung des Bandes (30; 100) aus flüssigkeitsdurchlässigem Material positioniert ist, mindestens einem weiteren Flüssigkeitseinlaß in die genannte erste Seite der Kissenformstation (20), welche dem ersten Einlaß (22; 116) aus der gegenüberliegenden Querseite zugekehrt ist, einer Vorrichtung (10, 12, 14, 16, 18) zum Zuführen feinverteilten Materials zu mindestens einem der Flüssigkeitseinlässe, einer Vorrichtung (28; 120; 122) zum Bereitstellen einer Druckdifferenz zwischen den ersten und zweiten Seiten der Kissenformstation (20), wodurch das feinverteilte Material veranlaßt wird, sich in die Öffnungen (26; 108) in dem Former (24) zu bewegen, um auf dem Band (30; 100) des flüssigkeitsdurchlässigen Materials abgelegt zu werden.

**15.** Vorrichtung nach Anspruch 14, bei welcher die Flüssigkeitseinlässe ständig offen sind.

**16.** Vorrichtung nach Anspruch 14, bei welcher die Flüssigkeitseinlässe Mittel zur Einstellung der Größe der weiteren Einlässe und/oder der Geschwindigkeiten des Flüssigkeitsstromes durch sie hindurch aufweisen.

**17.** Vorrichtung nach einem der Ansprüche 14 bis 16, wobei der Former (24) ein Ring ist.

**18.** Vorrichtung nach einem der Ansprüche 14 bis 17, wobei der Former (24) zwischen einer Innenkammer höheren Druckes und einer Außenkammer geringeren Druckes angeordnet ist.

**19.** Vorrichtung nach einem der Ansprüche 14 bis 18, wobei der Former (24) ein linearer Former ist.

**20.** Vorrichtung nach einem der Ansprüche 17 bis 19, wobei die Öffnungen (26; 108) C-förmig sind.

**Revendications**

**1.** Un procédé pour faire un produit comportant en tout ou en partie un coussin de matériaux finement divisés, ce procédé comprenant les étape consistant à :
(a) fournir un moule comportant une pluralité d'ouvertures qui y sont formées ;
(b) fournir une bande mobile continue de matériau perméable aux fluides sur un côté du moule pour boucher ce côté des ouvertures de façon perméable aux fluides;
(c) appliquer une différence de pression des fluides à travers le moule pour former un côté ayant une pression plus basse d'un côté du moule et un côté ayant une pression plus élevée de l'autre côté du moule, avec un écoulement de fluides au travers des ouvertures et du matériau perméable aux fluides bouchant les ouvertures ;
(d) introduire au moins deux flux d'entrée de fluide dans le côté ayant la pression plus élevée à partir des côtés opposés de façon transversale par rapport au sens de déplacement de la bande continue, au moins l'un desdits flux contenant des matériaux finement divisés qui y sont en suspension ;
(e) permettre aux matériaux finement divisés de passer dans les ouvertures et d'être déposés sur la bande continue de matériau perméable aux fluides ;
(f) et faire que la bande de matériau perméable aux fluides sur laquelle on trouve le coussin de matériaux finement divisés se sépare du moule.

**2.** Un procédé selon la revendication 1, dans lequel le moule est positionné dans une chambre semblable à une boîte et dans lequel deux flux de fluide entrent dans la chambre semblable à une boîte par les côtés opposés.

**3.** Un procédé selon la revendication 2, dans lequel les deux flux de fluide sont continus.

**4.** Un procédé selon la revendication 1, dans lequel le moule est positionné dans une chambre semblable à une boîte, l'intérieur de la chambre semblable à une boîte est divisé en au moins deux parties, et une paire de flux de fluide entre dans chaque partie.

**5.** Un procédé selon la revendication 4, dans lequel le passage du fluide à partir d'au moins l'une des parties est limité.

**6.** Un procédé selon l'une des revendications 2 à 4, dans lequel les deux flux de fluide contiennent des matériaux finement divisés qui y sont en suspension.

**7.** Un procédé selon l'une des revendications précédentes, dans lequel le fluide est de l'air.

**8.** Un procédé selon l'une des revendications 2 à 7, dans lequel les deux flux de fluide ont des vitesses d'écoulement différentes.

**9.** Un procédé selon l'une des revendications précédentes, dans lequel la bande de matériau perméable aux fluides est perméable à la vapeur mais substantiellement imperméable aux liquides.

**10.** Un procédé selon la revendication 9, dans lequel il y a de l'eau sur le côté du matériau perméable à la vapeur et imperméable aux liquides qui fait face au côté ayant la pression plus élevée du moule.

**11.** Un procédé selon la revendication 10, dans lequel la pression est ensuite appliquée sur le coussin pour former une mince couche densifiée.

**12.** Un procédé selon l'une des revendications 2 à 9, dans lequel il y a de l'adhésif sur le côté du matériau perméable aux fluides faisant face au côté ayant la pression plus élevée du moule.

**13.** Un procédé selon l'une des revendications précédentes, dans lequel une couche imperméable aux liquides est ensuite appliquée sur les coussins.

**14.** Un dispositif pour faire un produit comportant en tout ou en partie un coussin de matériaux finement divisés, ledit dispositif comprenant un poste de formation de coussins (20) comportant un moule (24) ayant une pluralité d'ouvertures (26, 108) qui y sont formées, un système adapté pour fournir une bande (30, 100) de matériau perméable aux fluides le long d'un côté du moule (24) pour boucher ce côté des ouvertures (26, 108) de façon perméable aux fluides, ledit moule (24) et ladite bande (30, 100) de matériau perméable aux fluides étant positionnés entre un premier côté du poste de formation de coussins (20) qui doit avoir une pression plus élevée qu'un second côté qui doit avoir une pression plus basse, une entrée de fluide (22, 116) vers le premier côté du poste de formation de coussins (20) et positionné transversalement par rapport au sens de déplacement de la bande (30, 100) du matériau perméable aux fluides, au moins une autre entrée de fluide vers ledit premier côté du poste de formation de coussins (20) et faisant face à la première entrée (22, 116) du côté transversal opposé, des moyens (10, 12, 14, 16, 18) pour fournir les matériaux finement divisés vers au moins l'une des entrées de fluide, des moyens (28, 120, 122) pour fournir une différence de pression entre les premier et second côtés du poste de formation de coussins (20), grâce à quoi les matériaux finement divisés se déplacent dans les ouvertures (26, 108) du moule (24) et se déposent sur la bande (30, 100) du matériau perméable aux fluides.

**15.** Un dispositif selon la revendication 14, dans lequel les autres entrées de fluide sont ouvertes en permanence.

**16.** Un dispositif selon la revendication 14, dans lequel les autres entrées de fluide ont des moyens pour adapter les tailles des autres entrées et/ou les vitesses d'écoulement du fluide au travers de celles-ci.

**17.** Un dispositif selon l'une des revendications 14 à 16, dans lequel le moule (24) est un anneau.

**18.** Un dispositif selon l'une des revendications 14 à 17, dans lequel le moule (24) est positionné entre une chambre interne ayant une pression plus élevée et une chambre externe ayant une pression plus basse.

**19.** Un dispositif selon l'une des revendications 14 à 18, dans lequel le moule (24) est un moule linéaire.

**20.** Un dispositif selon l'une des revendications 14 à 19, dans lequel les ouvertures (26, 108) sont en forme de C.

FIG 1

FIG 2

EP 0 270 976 B1

FIG 3

FIG 4

FIG 5

FIG 6

FIG 7

LP

HP3 ⊠         F1 ↑         ⊠ HP2

HP1

FIG 8a

W

FIG 8b

LP

HP3 ⊠         F1 ↑    F2         HP2

HP1

FIG 9a

FIG 9b                              W

FIG 10a

FIG 10b

FIG 11a

FIG 11b

FIG 12

FIG 13

FIG 14

FIG 15